# EUROPÄISCHE PATENTSCHRIFT

(11) **EP 3 202 672 B1**
(45) Veröffentlichungstag und Bekanntmachung des Hinweises auf die Patenterteilung: **25.09.2019**
(21) Anmeldenummer: 17153392.0
(22) Anmeldetag: 26.01.2017
(51) Int. Cl.: B65D 71/06, B65D 25/02, A61B 50/33, A61B 50/30, A61M 1/16, A61B 50/31, A61B 50/00, A61B 50/20, A61M 1/36, B65D 21/02, B65D 25/10

(54) **MEDIZINISCHE STERILVERPACKUNGSEINHEIT**
MEDICAL STERILE PACKAGING UNIT
UNITÉ D'EMBALLAGE STÉRILE À USAGE MÉDICAL

(30) Priorität: 05.02.2016 DE 102016102089
(43) Veröffentlichungstag der Anmeldung: 09.08.2017
(73) Patentinhaber: B. Braun Avitum AG, 34212 Melsungen (DE)
(72) Erfinder: DÖRING, Stefan, 01326 Dresden (DE); WIEGERS, Peter, 70599 Stuttgart (DE)
(74) Vertreter: Winter, Brandl, Fürniss, Hübner, Röss, Kaiser, Polte - Partnerschaft mbB

(56) Entgegenhaltungen:
- DE-U1- 9 107 610
- DE-U1-202004 003 964
- GB-A- 2 374 413
- GB-A- 2 525 694
- JP-A- 2011 006 082

## Beschreibung

Die Erfindung betrifft eine medizinische Sterilverpackungseinheit für ein steril verpacktes Medizinprodukt, insbesondere für ein Filtermodul/einen Dialysator (Filterpatrone, Filter für eine Blutbehandlungsmaschine, etc.)

Bei der Herstellung steriler Medizinprodukte, insbesondere Filtermodule/Dialysatoren, ist sicherzustellen, dass das Produkt bis zur Anwendung an einem Patienten oder Verwendung im Rahmen einer Behandlung steril bleibt. Dazu ist zu gewährleisten, dass entweder die Sterilbarriere an das Produkt gelegt wird oder dass die Verpackung eine Sterilbarriere gegenüber der Umgebung ausbildet, die unter Annahme realistischer Lagerbedingungen die auf dem Produkt angegebene Haltbarkeitsdauer (Shelflifetime) über unverletzt bleibt.

Filtermodule/Dialysatoren sind in ihrer äußeren Form ausschließlich im Hinblick auf fertigungs- und anwendungstechnische Erfordernisse ausgelegt. Es liegt auf der Hand, dass sich aus dieser Form besondere Anforderungen an die Filtermodul-/Dialysatorverpackung ableiten. Problematisch hinsichtlich der Verpackung sind insbesondere am Filtermodul/Dialysator standardisierte, abstehende und scharfkantige Konnektoren, Kanten am Filtermodul/Dialysator sowie an Schutzkappen (Protection Caps) des Filtermoduls/Dialysators.

Bekannte Verpackungen für Medizinprodukte, insbesondere für Filtermodule/Dialysatoren, bestehen primär aus einem Kunststoff- oder Aluminium-Schlauch- oder einem Siegelrandbeutel (als Primärverpackung) sowie aus einem Tray aus Kunststoff, Pappe oder Faserguss und ggf. einem Umkarton (als Sekundärverpackung). Insbesondere das Tray weist meist eine Form auf, die im Wesentlichen der Form des in der Primärverpackung verpackten Produkts entspricht, so dass eine Art Formschluss gegeben ist, mittels dem eine lagesichere Verpackung erstrebt wird.

Die britische Patentanmeldung GB 252 569 4 A offenbart eine stapelbare, verschachtelbare Endoskop Trays mit separater Aufnahme, Auskleidung und Abdeckung. Eine Endoskop-Trägerplatte umfasst eine Grundplatte mit Seitenwänden und hohlen Führungswänden, die von einer ersten Seite vorstehen, und Stützpfosten, die von der ersten oder zweiten Seite vorstehen und so angeordnet sind, dass die Trays in einer ersten Ausrichtung übereinander gestapelt werden können, wobei benachbarte Trays platzsparend dicht aneinander geschachtelt sind, oder in einer zweiten gedrehten Ausrichtung, bei der benachbarte Trays in einem größeren Abstand voneinander gestapelt werden.

Die britische Patentanmeldung GB 252 569 4 A offenbart ein Reservoirtray zur Herstellung und Übertragung von Reagenzien auf beispielsweise eine Mikrotiterplatte, umfassend einen Träger mit mindestens einer Reservoirkammer, dadurch gekennzeichnet, dass der Reservoirtray ferner Mittel zur Aufnahme mindestens einer Komponente eines Assay-Kits umfasst, so dass die mindestens eine Komponente des darin enthaltenen Assay-Kits durch die Reservoirschale während der Handhabung oder des Transports geschützt ist. Ebenfalls beschrieben ist ein Assay-Kit, verpackt in einer Box, bestehend aus einem Behälter und einem Reservoir-Tray.

Einige Medizinprodukte, insbesondere Filtermodule/Dialysatoren, müssen ggf. unter sauerstofffreien Bedingungen sterilisiert werden. Das bedeutet, dass zum Zeitpunkt der Sterilisierung das Innere der Primärverpackung (absolut) sauerstofffrei sein muss. Dies wird in aller Regel durch Absorption des Sauerstoffs durch einen geeigneten Träger, einen sogenannten Getter, realisiert. Das Trägermaterial kann dabei beispielsweise Eisenpulver oder ein Polymer sein. Der Absorber kann als sogenanntes Sachet der Primärverpackung zugeführt werden oder in der Struktur des Verpackungsmaterials (Folie) enthalten sein.

Es ist ein beträchtlicher Nachteil, dass eine Bindung von molekularem Sauerstoff im abgeschlossenen System der Primärverpackung eine Volumenreduzierung bzw. einen Unterdruck (bei einer formunveränderlichen Umgebung) zur Folge hat. Bekannte Verpackungssysteme sind nicht formstabil, folglich reduziert sich ihr Volumen entsprechend der Sauerstoffbindung nach Verschließen der Verpackung in nicht kontrollierbarer Weise. Eine solche Volumenreduzierung der Primärverpackungen ermöglicht Relativbewegungen zwischen verpackten Filtermodulen/Dialysatoren untereinander innerhalb der Sekundärverpackung sowie zwischen verpackten Filtermodulen/Dialysatoren und der Sekundärverpackung, wobei diese Relativbewegungen wiederum zu Beschädigung der Sterilbarriere führen können. Herkömmliche Trays respektive Umkartons können nicht auf eine solche Volumenänderung oder -reduzierung reagieren, da sie starr sind.

Ein weiterer Nachteil bekannter Verpackungssysteme ist, dass diese in der Zahl der Produkte, die sie beinhalten, fix sind. Dieser Umstand wiegt umso schwerer, als Filtermodule/Dialysatoren in unterschiedlichen Baugrößen auf den Markt gebracht werden, um unterschiedlichen Blutvolumina einzelner Patienten zu entsprechen. In aller Regel wird dabei (aus fertigungs-und logistischen Gründen) weniger die Länge der Baugruppe als vielmehr deren Durchmesser variiert. Mit wachsender Membranfläche nimmt der Durchmesser der Baugruppen zu. In aller Regel sind dann durchmesserspezifische Trays und Umkartons erforderlich.

Schließlich werden im Klinikalltag bestimmte Medizinprodukte wie Filtermodule/Dialysatoren vom Behandlungsraum getrennt gelagert. Klinikpersonal muss die Produkte daher regelmäßig aus einem Lager in einen Behandlungsraum transportieren. Bislang gab es dazu keine Hilfsmittel. Das Personal war auf eigene Lösungen angewiesen, diese Transportaufgabe zu meistern. Folge waren zuweilen unzweckmäßige, die Sterilbarriere der Verpackung oftmals in Mitleidenschaft ziehende Transportmethoden.

Ausgehend von dem vorstehend beschriebenen Stand der Technik liegt der vorliegenden Erfindung die Aufgabe zugrunde, die zuvor angeführten Nachteile zu beseitigen, insbesondere eine Sterilverpackungseinheit für Medizinprodukte, vorzugsweise Filtermodule bereit zu stellen, mittels der Beschädigungen der Sterilbarriere infolge von Relativbewegungen zwischen Verpackungen, die durch unkontrollierte Volumenreduzierung möglich werden, minimiert oder vorzugsweise verhindert werden können. Die Verpackung selbst soll vorzugsweise gegenüber Volumenreduzierung unempfindlich sowie kostengünstig und formstabil sein. Schließlich soll vorzugsweise die Sterilverpackungseinheit an die Größe von darin verpackten Medizinprodukten anpassbar und hinsichtlich der Anzahl der verpackten Medizinprodukte weitgehend variabel sein. Herstellungskosten und Verpackungsaufwand sollen weiter vorzugsweise minimiert werden und Produktschutz auch außerhalb einer Umverpackung sichergestellt sein.

Nach der Erfindung wird diese Aufgabe gelöst durch eine (medizinische) Sterilverpackungseinheit mit den Merkmalen des Anspruchs 1. Bei dem Medizinprodukt kann es sich insbesondere um einen Dialysator oder ein Filtermodul handeln. Dies kann insbesondere einen im Wesentlichen zylinderförmigen Mittenabschnitt und einen endseitig des Mittenabschnitts ausgebildeten Filtermodul-/Dialysatoranschluss aufweisen.

Hintergrund der Erfindung ist, in beliebiger Art steril (primär) verpackte Medizinprodukte, insbesondere Filtermodule oder Dialysatoren, einer schachtelbaren Sekundärverpackung zuzuordnen, die aufgrund Ihres Designs gleichzeitig als Tray und Umkarton dient. Neben einer Reduzierung von Verpackungsaufwand und Kosten wird beim Anwender ein komfortables Handling der Baugruppen ermöglicht: Die Trays können ohne Umreifung zum Beispiel in Regale eingelagert werden und bieten den Produkten auch dort und beim Transport beim Anwender umfassenden Schutz. Lagerschäden werden somit weitgehend vermieden. Darüber hinaus sind die Trays als Transportmöglichkeit durch den Anwender verwendbar, etwa beim Tragen aus dem Lager zum Therapieplatz. Ein weiterer Vorteil ist, dass das Tray als ein einziges Element mehrere Funktionen übernimmt, wofür im Stand der Technik mehrere Elemente oder Einheiten erforderlich waren. Es besitzt nämlich sowohl Tray- als auch Sekundärverpackungsfunktion. Durch Stapelung einer mehr oder weniger großen Zahl von Trays kann eine mehr oder weniger große Zahl von Filtermodulen/Dialysatoren zu einer Verpackungseinheit kombiniert werden (bisher in einem Karton mit fixer Anzahl an verpackten Einheiten) und als eine Verpackungseinheit ausgeliefert werden. Das Tray funktioniert als sicheres Transporthilfsmittel für eine handliche Zahl von Filtermodulen/Dialysatoren. Des Weiteren kann durch die Erfindung Verpackungsmüll reduziert werden. Herkömmliche Verpackungssysteme sicheren die verpackten Medizinprodukte in der Regel in einem Karton durch Zwischenlagen oder Trays. Nach Entleerung sind alle Karton, Zwischenlagen und Trays zu entsorgen. Das erfindungsgemäße Verpackungssystem reduziert das Müllvolumen um den Karton, da es ohne einen solchen auskommt. Schließlich vereinfacht der Tray die Handhabung der darauf gelagerten Filtermodule/Dialysatoren insbesondere nach deren Entpacken. So ist der Tray dafür ausgebildet (insbesondere hinsichtlich seiner Verwindungssteifigkeit), quasi als Tablett für die entpackten Filtermodule/Dialysatoren zu dienen, auf dem die Filtermodule/Dialysatoren frei entnehmbar aufgelagert sein können.

Vorteilhafte Ausführungsformen der Erfindung sind in den Unteransprüchen beansprucht und werden nachfolgend näher erläutert.

Die Primärverpackung kann insbesondere einen Getter zur Bindung von in der Primärverpackung vorliegendem molekularem Sauerstoff aufweisen, sodass nachfolgend eine Sterilisation durch Strahlen (Gamma) möglich ist.

Bei der Primärverpackung handelt es sich vorzugsweise um eine Blisterverpackung, insbesondere um einen Hart-Hart- oder Hart-Weich-Blister. Sie kann ein eine Aufnahme für ein Medizinprodukt ausbildendes Kunststoffformteil als Unterteil und ein die Aufnahme verschließendes Oberteil aufweisen, wobei das Oberteil hermetisch dicht am Unterteil festgelegt (verschweißt, verklebt, etc.) ist.

Die Banderole besteht vorzugsweise aus Papier, Packpapier, Karton, Folie, Kunststofffolie oder einem Gewebematerial. Sie kann insbesondere elastisch sein. Auf diese Weise kann sie Umfangsänderungen des einen Trays bzw. der von ihr umhüllten Trays mit den darin aufgenommenen primärverpackten Medizinprodukten durch elastische Verformung (Längung/Kürzung) folgen.

Vorzugsweise weist das Tray einen Boden und um diesen herum angeordnete Seitenwände auf. Die Mehrzahl an Aufnahmen ist, zum Beispiel in Form von Vertiefungen, Wellen, etc., im oder am Boden oder durch den Boden ausgebildet. Insbesondere kann der Boden wellenförmig mit Vertiefungen und Erhebungen ausgebildet sein, derart, dass diese sowohl an der Oberseite des Bodens als auch an der Unterseite des Bodens ausgebildet sind.

Bei einer Ausführungsform sind die Seitenwände des Tray konisch angeordnet oder ausgebildet. Auf diese Weise sind die Seitenwände an der Oberseite des Tray weiter voneinander beabstandet, als an der Unterseite des Tray und/oder als am Boden. Herkömmliche Trays mit Umkartons können nicht auf eine Volumenreduzierung reagieren, da sie starr sind. Die genannte Ausführungsform kann diesen Nachteil überwinden, da die Trays infolge ihrer konischen Gestalt ineinandergreifen können und einer Volumenreduzierung, z. B. der darin jeweils eingelegten Primärverpackungen, zum Beispiel infolge einer Bindung von molekularem Sauerstoff in der Primärverpackung nachfolgen können. Die Trays lassen sich ineinanderstecken, so dass den unterschiedlichen Außendurchmessern der unterschiedlichen Primärverpackungen mit nur einem universellen Tray entsprochen werden kann. Man kann auch sagen, dass durch ein Ineinanderstapeln von Trays eine Anpassung des Verpackungssystems an die Produktgrößen erfolgt. Anders ausgedrückt, sind aufeinander gestapelte Trays zueinander in Höhenrichtung weiterhin positionierbar, so dass zum Beispiel Volumenänderungen innerhalb der Primärverpackungen Rechnung getragen werden kann.

Besonders guter Schutz der primärverpackten Medizinprodukte kann dadurch erzielt werden, dass die Seitenwände die in den Aufnahmen des Tray angeordneten primärverpackten Medizinprodukte überragen. Eine weitere Möglichkeit dazu ist, dass das Tray Eckenabschnitte, insbesondere stabilisierende Eckenabschnitte, aufweist, die über die Seitenwände hinausragen.

Eine besonders gute Anpassbarkeit der Sterilverpackungseinheit kann erzielt werden, indem eine Mehrzahl von Trays mit darin aufgenommenen primärverpackten Medizinprodukten übereinander angeordnet ist, wobei der Boden eines Trays auf den primärverpackten Medizinprodukten des darunter angeordneten Trays aufliegt und/oder die konisch angeordneten Seitenwände eines Trays auf den konisch angeordneten Seitenwänden des darunter angeordneten Trays aufliegen.

Das Medizinprodukt kann insbesondere ein in der Primärverpackung steril verpackter Filtermodul/Dialysator mit einem im Wesentlichen zylinderförmigen Mittenabschnitt und einem endseitig des Mittenabschnitts ausgebildeten Filtermodul-/Dialysatoranschluss, vorzugsweise mit jeweils einem endseitig des Mittenabschnitts ausgebildeten Filtermodul-/ Dialysatoranschluss, sein. Die Primärverpackung kann insbesondere eine Blisterverpackung mit einem eine Aufnahme für das Filtermodul/den Dialysator ausbildenden Kunststoffformteil als Unterteil und einer mit diesem einen Aufnahmeraum für das Filtermodul/den Dialysator ausbildenden Oberfolie sein. Die Oberfolie kann hermetisch dicht am Unterteil festgelegt sein und das Filtermodul/den Dialysator zumindest abschnittsweise durch Formschluss fixieren, insbesondere gegenüber dem Unterteil oder zwischen sich und dem Unterteil fixieren. Die Oberfolie kann sich insbesondere zumindest an den Mittenabschnitt des Filtermoduls/Dialysators anschmiegen. Zwischen Unterteil und Oberfolie ist dann nur eine geringe Menge an Luft und damit molekularem Sauerstoff enthalten, was für eine Sterilisierung des in der Primärverpackung verpackten Filtermoduls/Dialysators vorteilhaft ist. Im Falle einer Sterilisierung mittels Strahlen/Gamma-Strahlung, wofür in der verschlossenen und abgedichteten Verpackung enthaltener molekularer Sauerstoff zu entfernen ist, fällt eine Volumenreduzierung infolgedessen in vorteilhafter Weise gering aus.

Die Primärverpackung ist vorzugsweise derart ausgebildet, dass das Medizinprodukt, insbesondere das Filtermodul/der Dialysator, darin sicher und fixiert gehalten oder lagepositioniert ist. Nach einer Ausführungsform kann das durch Definition eines Bereiches innerhalb der Verpackung erfolgen, an dem die Verpackung, insbesondere das Formteil sich infolge einer Volumenreduzierung ohne Beeinträchtigung der Gesamtstabilität und der grundsätzlichen Form der Verpackung verformen kann. Ohne Beeinträchtigung der grundsätzlichen Form der Verpackung bedeutet in diesem Sinne, dass bestimmte Außenbereiche der Verpackung, über die sie zum Beispiel an anderen Primärverpackungen oder an einer Umverpackung abgestützt ist, sich nicht verformen. Ebenfalls im Wesentlichen unverformt bleiben Innenabschnitte der Verpackung, insbesondere des Formteils, über die oder an denen das Filtermodul/der Dialysator gehalten, gelagert oder abgestützt ist.

Eine Besonderheit der Erfindung ist, das das Unterteil so gestaltet sein kann, dass es das Medizinprodukt, insbesondere das Filtermodul/denDialysator, sicher aufnimmt. Vorzugsweise ist das Unterteil als eine Art Tablett ausgebildet, dass eine Vertiefung für das Medizinprodukt besitzt, dessen Höhe jedoch deutlich geringer als die Abmessungen des Medizinprodukts quer zu dessen Axialrichtung ist. Vorzugsweise beträgt die Tiefe der im Unterteil ausgestalteten Aufnahme weniger als die Hälfte des Durchmessers des Medizinprodukts, besonders bevorzugt weniger als ein Drittel des Durchmessers des Medizinprodukts, noch bevorzugter weniger als ein Viertel des Durchmessers des Medizinprodukts. Auf diese Weise wird einfach sichergestellt, dass das Medizinprodukt zum größeren Teil von der Oberfolie umhüllt und durch diese lagefixiert ist. Des Weiteren kann das Unterteil derart gestaltet sein, dass ein Verschließen von Filtermodul-/Dialysatoranschlüssen durch die Oberfolie verhindert wird bzw. nicht möglich ist. Diese müssen im Falle einer Strahlen(Gamma)-Sterilisierung zwingend freibleiben, um eine Bewegung von Sauerstoff-Molekülen aus dem Faserbündel des Filtermoduls/Dialysators zu ermöglichen. Insbesondere kann die Oberfolie (eng) an dem zylinderförmigen Mittenabschnitt des Filtermoduls/Dialysators anliegen und die jeweils endseitig des Mittenabschnitts ausgebildeten Filtermodul-/Dialysatoranschlüsse des Filtermoduls/Dialysators nicht kontaktieren. Vorzugsweise ist die Oberfolie eine Schrumpffolie.

Das feste Unterteil der Blisterkombination kann dabei einen Rand aufweisen. Die Oberfolie kann an dem vorzugsweise kontinuierlich umlaufenden Rand des Unterteils aufgesiegelt sein. Der Rand kann insbesondere formschlüssig zum Tray sein und an dessen Seitenwänden anliegen und somit Relativbewegungen zwischen Primärverpackung und Tray einschränken bzw. verhindern.

Das Unterteil bildet vorzugsweise beiderseits endseitig des Mittenabschnitts des Filtermoduls/Dialysators jeweils eine gegenüber der Außenkontur des Filtermoduls/Dialysators, insbesondere gegenüber den Filtermodul-/Dialysatoranschlüssen, aufgeweitete Anschlussaufnahme aus. In diese können die Filtermodul-/Dialysatoranschlüsse aufgenommen sein, so dass diese in vorteilhafter Weise weder durch das Unterteil noch durch die Oberfolie verschlossen sind und in der jeweiligen Anschlussaufnahme offen und strömungstechnisch mit dem Aufnahmeraum, insbesondere mit dem Anschlussaufnahmevolumen, verbunden sind.

Das Tray ist vorzugsweise derart ausgebildet, dass mit dem primärverpackten Medizinprodukt Formschluss in axialer Richtung und/oder in tangentialer Richtung besteht. Die Aufnahmen können in Form von Vertiefungen, vorzugsweise teilzylinderförmige Vertiefungen, im Boden vorliegen. Jeweils eine Primärverpackung ist formschlüssig in eine Vertiefung einlegbar bzw. eingelegt.

Zusammenfassend kann man sagen, dass die Erfindung eine Möglichkeit beschreibt, steril verpackte Medizinprodukte, insbesondere Filtermodule/Dialysatoren unterschiedlicher Außenabmessungen (Durchmesser) in einem universellen Tray zu verpacken. Die Trays werden dabei größenabhängig ineinandergesteckt und zum Versand lediglich mit einer Banderole, bspw. aus Papier, umreift. Das Tray dient darüber hinaus als Lager- und Transportstabilisierung beim Anwender.

Mit der Erfindung werden unter anderem die folgenden Vorteile erzielt:
- Einsparung von größenspezifischen Zwischenlagen (Trays) und Umkartons, was zu einer Senkung von Herstellkosten führt,
- Verbesserte Möglichkeit der Automatisierung (Handling), was zu einer Senkung von Herstellkosten führt,
- Ermöglichung einer hohe Anzahl von Medizinprodukten im Karton, was zu einer Senkung von Logistikkosten führt,
- Einschränkung von diversen Relativbewegungen zwischen Primärverpackung und Umverpackung durch nachgebende Steckverbindung zwischen den Trays,
- Möglichkeit der Variation der Zahl der Filtermodule/Dialysatoren in der Verpackungseinheit,
- vereinfachter und sicherer Transport bzw. Lagerung der Filtermodule/Dialysatoren in der Klinik und
- Reduzierung von Verpackungsmüll.

Die Erfindung wird im Folgenden anhand beispielhafter, nicht einschränkender und in den angehängten Figuren gezeigter Ausführungsformen näher erläutert. Dabei zeigt:
Figur 1 eine schematische perspektivische Darstellung eines Tray einer Sterilverpackungseinheit,
Figur 2 das Tray der Figur 1 in einer Schnittansicht quer zur Axialrichtung,
Figur 3 eine perspektivische Ansicht einer Sterilverpackungseinheit in einer ersten Ausführungsform,
Figur 4 eine perspektivische Ansicht einer Sterilverpackungseinheit in einer zweiten Ausführungsform mit Trays der Figuren 1 und 2.

Der nachfolgend verwendete Ausdruck "Dialysator" ist dabei so zu verstehen dass auch andere Arten von Filtermodulen mit eingeschlossen sind.

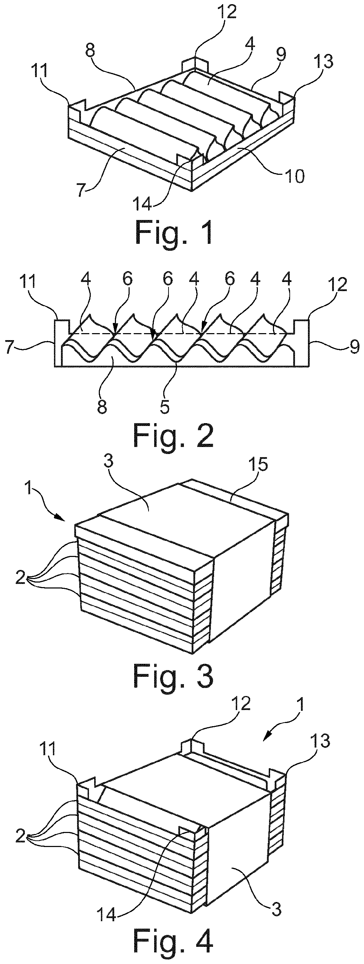

Figur 4 zeigt eine Sterilverpackungseinheit 1 mit vier Trays 2, die in Auflagerrichtung übereinander gestapelt sind und die von einer gemeinsamen Banderole 3 umhüllt und zusammengehalten sind. Die Banderole 3 umhüllt die Trays 2 (im Wesentlichen) vollumfänglich und bildet so quasi die Sekundärverpackung. Eines der Trays 2 ist in den Figuren 1 und 2 gezeigt. Es beinhaltet vorzugsweise fünf Dialysator-Primärverpackungen 4, jeweils bestehend aus Primärverpackungen, in denen jeweils ein darin verpackter Dialysator als Medizinprodukt (in den Figuren nicht gezeigt) steril verpackt ist.

Jedes Tray 2 weist einen Boden 5 auf. Dieser ist vorzugsweise gewellt ausgebildet, so dass an seiner Oberseite Aufnahmen 6 für die primärverpackten Dialysatoren ausgebildet sind. Der Boden 5 ist von Seitenwänden 7, 8, 9, 10 umgeben, die mittels Eckabschnitten 11, 12, 13, 14 jeweils miteinander verbunden sind, wodurch ein Rahmen gebildet wird. Insbesondere Figur 2 zeigt, dass die Eckabschnitte 11, 12, 13, 14 höher sind als die im Tray 2 aufgenommenen primärverpackten Produkte 4. Dadurch entsteht quasi eine stapelbare Dialyatoren-Steige.

Bei der Ausführungsform der Figur 3 ist auf dem oberen (obersten) Tray 2 des Tray-Stapels ein Deckel 15 angeordnet, der die primärverpackten Dialysatoren abdeckt und der ebenfalls von der Banderole 3 zusätzlich umhüllt ist. Die Figuren 3 und 4 zeigen die Sterilverpackungseinheit 1 in einem Zustand verpackt mit der Banderole 3 und bereit zum Versand. Die Figuren 1 und 2 zeigen die Sterilverpackungseinheit 1 in einem Zustand bei entfernter Banderole 3 beispielsweise zum Lagern bei einem Anwender und zum Transport vom Lager zu einer Anwendung.

### Bezugszeichen

- 1: Sterilverpackungseinheit
- 2: Tray
- 3: Banderole
- 4: Primärverpackung
- 5: Boden
- 6: Aufnahme
- 7: Seitenwand
- 8: Seitenwand
- 9: Seitenwand
- 10: Seitenwand
- 11: Eckabschnitt
- 12: Eckabschnitt
- 13: Eckabschnitt
- 14: Eckabschnitt
- 15: Deckel

## Patentansprüche

1. Sterilverpackungseinheit (1) mit einem steril verpackten Medizinprodukt, insbesondere einem Filtermodul, aufweisend
eine Primärverpackung (4) mit dem darin steril und hermetisch dicht verpackten Medizinprodukt,
eine Mehrzahl von schachtelbaren, übereinander angeordneten Trays (2) jeweils mit einer Mehrzahl an Aufnahmen (6) für jeweils ein in der Primärverpackung (4) verpacktes Medizinprodukt,
**dadurch gekennzeichnet, dass**
die Sterilverpackungseinheit (1) mindestens eine die Mehrzahl an Trays (2) mit den darin aufgenommenen primärverpackten Medizinprodukten umfassende Banderole (3) aufweist.

2. Sterilverpackungseinheit (1) nach Anspruch 1, **dadurch gekennzeichnet, dass** die Primärverpackung (4) mindestens einen Getter zur Bindung von in der Primärverpackung (4) vorliegendem molekularem Sauerstoff aufweist.

3. Sterilverpackungseinheit (1) nach Anspruch 1 oder 2, **dadurch gekennzeichnet, dass** die Primärverpackung (4) eine Blisterverpackung mit einem eine Aufnahme für ein Medizinprodukt ausbildenden Kunststoffformteil als Unterteil und einem die Aufnahme verschließenden Oberteil ist, wobei das Oberteil hermetisch dicht am Unterteil festgelegt ist.

4. Sterilverpackungseinheit (1) nach einem der vorstehenden Ansprüche,
**dadurch gekennzeichnet, dass** die Banderole (3) aus Papier, Packpapier, Karton, Folie, Kunststofffolie oder einem Gewebematerial besteht.

5. Sterilverpackungseinheit (1) nach einem der vorstehenden Ansprüche,
**dadurch gekennzeichnet, dass** die Banderole (3) elastisch ist und Umfangsänderungen des zumindest einen Trays (2) mit den darin aufgenommenen primärverpackten Medizinprodukten durch elastische Verformung folgen kann.

6. Sterilverpackungseinheit (1) nach einem der vorstehenden Ansprüche,
**dadurch gekennzeichnet, dass** das Tray (2) einen Boden (5) und um diesen herum angeordnete Seitenwände (7, 8, 9, 10) aufweist, wobei die Anzahl an Aufnahmen (6) im oder am Boden (5) oder durch den Boden (5) ausgebildet ist.

7. Sterilverpackungseinheit (1) nach Anspruch 6, **dadurch gekennzeichnet, dass** der Boden (5) wellenförmig mit Vertiefungen (6) und Erhebungen ausgebildet ist, derart, dass diese sowohl an der Oberseite des Bodens (5) als auch an der Unterseite des Bodens (5) ausgebildet sind.

8. Sterilverpackungseinheit (1) nach Anspruch 6 oder 7, **dadurch gekennzeichnet, dass** die Seitenwände (7, 8, 9, 10) des Tray (2) konisch angeordnet oder ausgebildet sind, so dass die Seitenwände (7, 8, 9, 10) an der Oberseite des Tray (2) weiter voneinander beabstandet sind, als an der Unterseite des Tray (2) und/oder als am Boden (5).

9. Sterilverpackungseinheit (1) nach einem der vorstehenden Ansprüche 5 bis 8, **dadurch gekennzeichnet, dass** die Seitenwände (7, 8, 9, 10) die in den Aufnahmen des Tray (2) angeordneten primärverpackten Medizinprodukte überragen.

10. Sterilverpackungseinheit (1) nach einem der vorstehenden Ansprüche 5 bis 9, **dadurch gekennzeichnet, dass** das Tray (2) Eckenabschnitte (11, 12, 13, 14), insbesondere stabilisierende Eckenabschnitte (11, 12, 13, 14), aufweist, die über die Seitenwände (7, 8, 9, 10) hinausragen.

11. Sterilverpackungseinheit (1) nach einem der vorstehenden Ansprüche 5 bis 10, **dadurch gekennzeichnet, dass** eine Mehrzahl von Trays (2) mit darin aufgenommenen primärverpackten Medizinprodukten übereinander angeordnet ist, wobei der Boden (5) eines Trays (2) auf den primärverpackten Medizinprodukten des darunter angeordneten Trays (2) aufliegt und/oder die konisch angeordneten Seitenwände (7, 8, 9, 10) eines Trays (2) auf den konisch angeordneten Seitenwänden (7, 8, 9, 10) des darunter angeordneten Trays (2) aufliegen.

12. Sterilverpackungseinheit (1) nach einem der vorstehenden Ansprüche,
**dadurch gekennzeichnet, dass** das Medizinprodukt ein Filtermodul ist, wobei das Filtermodul einen im Wesentlichen zylinderförmigen Mittenabschnitt und einen endseitig des Mittenabschnitts ausgebildeten Filtermodulanschluss aufweist.

## Claims

1. Sterile packaging unit (1) with a sterile-packaged medical product, in particular a filter module, comprising
a primary packaging (4) with the sterile-packaged and hermetically sealed medical product inside it,
a plurality of nestable trays (2) on top of each other, each with a plurality of inserts (6) for each of the medical products packaged in the primary packaging (4), and
**characterized in that**
the sterile packaging unit (1) comprises at least one sleeve (3) which wraps around the plurality of trays (2) with the primary-packaged medical products contained in them.

2. Sterile packaging unit (1) according to Claim 1, **characterized in that** the primary packaging (4) comprises at least one getter to bind molecular oxygen in the primary packaging (4).

3. Sterile packaging unit (1) according to Claim 1 or 2, **characterized in that** the primary packaging (4) is a blister packaging with a plastic moulded part forming an insert for a medical product as its lower section and an upper section which closes the insert, wherein the upper section is fixed onto the lower section so as to be hermetically sealed.

4. Sterile packaging unit (1) according to one of the preceding claims, **characterized in that** the sleeve (3) is made of paper, packing paper, cardboard, foil, plastic foil or a fabric material.

5. Sterile packaging unit (1) according to one of the preceding claims, **characterized in that** the sleeve (3) is elastic and can follow changes in dimension of the at least one tray (2) with the primary packaged medical products contained in it by means of elastic deformation.

6. Sterile packaging unit (1) according to one of the preceding claims, **characterized in that** the tray (2) comprises a base (5) and side walls (7, 8, 9, 10) arranged around the base (5), whereby the number of inserts (6) are formed in or on the base (5) or by the base (5).

7. Sterile packaging unit (1) according to Claim 6, **characterized in that** the base (5) is configured to be wave-shaped with recesses (6) and protrusions in such a way that the latter are formed both on the upper side of the base (5) and on the lower side of the base (5).

8. Sterile packaging unit (1) according to Claim 6 or 7, **characterized in that** the side walls (7, 8, 9, 10) of the tray (2) are arranged or configured in a conical fashion so that the side walls (7, 8, 9, 10) on the upper side of the tray (2) are spaced further apart than on the lower side of the tray (2) and/or on the base (5).

9. Sterile packaging unit (1) according to one of the preceding claims 5 to 8, **characterized in that** the side walls (7, 8, 9, 10) extend above the primary packaged medical products arranged in the inserts of the tray (2).

10. Sterile packaging unit (1) according to one of the preceding claims 5 to 9, **characterized in that** the tray (2) comprises corner sections (11, 12, 13, 14), in particular stabilizing corner sections (11, 12, 13, 14), which protrude beyond the side walls (7, 8, 9, 10).

11. Sterile packaging unit (1) according to one of the preceding claims 5 to 10, **characterized in that** a plurality of trays (2) are arranged on top of each other, with the primary packaged medical products inserted in them, wherein the base (5) of a tray rests on the primary packaged medical products in the tray (2) below it and/or the conically arranged side walls (7, 8, 9, 10) of a tray (2) rest on the conically arranged side walls (7, 8, 9, 10) of the tray below it.

12. Sterile packaging unit (1) according to one of the preceding claims, **characterized in that** the medical product is a filter module, wherein the filter module comprises a substantially cylindrical central section and a filter module connection formed at the end of the central section.

## Revendications

1. Unité de conditionnement stérile (1) avec un produit médical à conditionnement stérile, en particulier module filtrant, présentant
un conditionnement primaire (4) avec le produit médical conditionné dans celui-ci de manière stérile et de manière étanche hermétiquement,
une multitude de plateaux (2) pouvant être empilés, disposés les uns par-dessus les autres respectivement avec une multitude de logements (6) pour respectivement un produit médical conditionné dans le conditionnement primaire (4),
**caractérisée en ce que**
l'unité de conditionnement stérile (1) présente au moins une banderole (3) comprenant la multitude de plateaux (2) avec les produits médicaux conditionnés de manière primaire logés dans ceux-ci.

2. Unité de conditionnement stérile (1) selon la revendication 1, **caractérisée en ce que** le conditionnement primaire (4) présente au moins un piège à gaz pour lier de l'oxygène moléculaire présent dans le conditionnement primaire (4).

3. Unité de conditionnement stérile (1) selon la revendication 1 ou 2, **caractérisée en ce que** le conditionnement primaire (4) est un conditionnement blister avec une partie moulée en matière plastique réalisant un logement pour un produit médical en tant que partie inférieure et une partie supérieure fermant le logement, dans laquelle la partie supérieure est fixée de manière étanche hermétiquement au niveau de la partie inférieure.

4. Unité de conditionnement stérile (1) selon l'une quelconque des revendications précédentes, **caractérisée en ce que** la banderole (3) est constituée de papier, de papier d'emballage, de carton, de film, de film en plastique ou d'un matériau en tissu.

5. Unité de conditionnement stérile (1) selon l'une quelconque des revendications précédentes, **caractérisée en ce que** la banderole (3) est élastique et des modifications de périphérie de l'au moins un plateau (2) avec les produits médicaux conditionnés de manière primaire logés dans celui-ci peuvent être entraînées par une déformation élastique.

6. Unité de conditionnement stérile (1) selon l'une quelconque des revendications précédentes, **caractérisée en ce que** le plateau (2) présente un fond (5) et des parois latérales (7, 8, 9, 10) disposées tout autour de celui-ci, dans laquelle le nombre de logements (6) est réalisé dans ou au niveau du fond (5) ou par le fond (5).

7. Unité de conditionnement stérile (1) selon la revendication 6, **caractérisée en ce que** le fond (5) est réalisé de manière à présenter une forme ondulée avec des creux (6) et des parties surélevées de telle manière que ceux-ci sont réalisés à la fois au niveau du côté supérieur du fond (5) et au niveau du côté inférieur du fond (5).

8. Unité de conditionnement stérile (1) selon la revendication 6 ou 7, **caractérisée en ce que** les parois latérales (7, 8, 9, 10) du plateau (2) sont disposées ou sont réalisées de manière conique de sorte que les parois latérales (7, 8, 9, 10) sont espacées les unes des autres davantage au niveau du côté supérieur du plateau (2) qu'au niveau du côté inférieur du plateau (2) et/ou qu'au niveau du fond (5).

9. Unité de conditionnement stérile (1) selon l'une quelconque des revendications 5 à 8, **caractérisée en ce que** les parois latérales (7, 8, 9, 10) dépassent des produits médicaux conditionnés de manière primaire, disposés dans les logements du plateau (2).

10. Unité de conditionnement stérile (1) selon l'une quelconque des revendications 5 à 9, **caractérisée en ce que** le plateau (2) présente des sections de coin (11, 12, 13, 14), en particulier des sections de coin (11, 12, 13, 14) de stabilisation, qui s'avancent au-delà des parois latérales (7, 8, 9, 10).

11. Unité de conditionnement stérile (1) selon l'une quelconque des revendications 5 à 10, **caractérisée en ce qu'**une multitude de plateaux (2) avec des produits médicaux conditionnés de manière primaire logés dans ceux-ci sont disposés les uns par-dessus les autres, dans laquelle le fond (5) d'un plateau (2) repose sur les produits médicaux conditionnés de manière primaire du plateau (2) disposé en dessous et/ou les parois latérales (7, 8, 9, 10), disposées de manière conique, d'un plateau (2) reposent sur les parois latérales (7, 8, 9, 10), disposées de manière conique, du plateau (2) disposé en dessous.

12. Unité de conditionnement stérile (1) selon l'une quelconque des revendications précédentes, **caractérisée en ce que** le produit médical est un module filtrant, dans laquelle le module filtrant présente une section centrale sensiblement de forme cylindrique et un raccord de module filtrant réalisé côté extrémité de la section centrale.
